# EUROPEAN PATENT APPLICATION

(11) **EP 3 978 626 A1**
(43) Date of publication of application: **06.04.2022**
(21) Application number: 20199844.0
(22) Date of filing: 02.10.2020
(51) Int. Cl.: C12Q 1/6865

(54) **METHOD AND MEANS FOR GENERATING TRANSCRIBED NUCLEIC ACIDS**

(71) Applicant: Lexogen GmbH, 1030 Vienna (AT)
(72) Inventor: SURVILA, Mantas, 1030 Wien (AT); MOLL, Pamela, 1200 Wien (AT); SEITZ, Alexander, 1140 Wien (AT)
(74) Representative: SONN Patentanwälte OG

(57) **Abstract**

The present invention relates to a method of generating transcribed nucleic acids, comprising the steps of providing a nucleic acid template, hybridizing an oligonucleotide probe to the nucleic acid template, wherein said oligonucleotide probe comprises a complementary part that hybridizes to the nucleic acid template and a non-complementary part in 5' direction of the complementary part that does not hybridize to the nucleic acid template and comprises a sequence of a transcription promoter, hydrolysing a 3' part of the nucleic acid template that is in 3' direction to a part of the nucleic acid template that hybridizes to the oligonucleotide probe and wherein said 3' part is not hybridized to the oligonucleotide probe, extending the nucleic acid template with nucleic acids complementary to the non-complementary part of the oligonucleotide probe, thereby generating a duplex of the sequence of the transcription promoter in sequence with the nucleic acid template, transcribing the nucleic acid template with a transcriptase that binds the duplex of the sequence of the transcription promoter, thereby generating the transcribed nucleic acids; and kits and nucleic acids for performing such a method.

SEQUENCE LISTING

<110>  LEXOGEN GMBH

<120>  Method and means for generating transcribed nucleic acids

<130>  R 76947

<160>  9

<170>  PatentIn version 3.5

<210>  1
<211>  45
<212>  DNA
<213>  Artificial Sequence

<220>
<223>  DNA probe

<400>  1
ctaatacgac tcactatagg gagatttttt tttttttttt ttttv                        45


<210>  2
<211>  45
<212>  DNA
<213>  Artificial Sequence

<220>
<223>  DNA probe

<400>  2
ctaatacgac tcactatagg gagatttttt tttttttttt ttttv                        45


<210>  3
<211>  68
<212>  DNA
<213>  Artificial Sequence

<220>
<223>  DNA probe

<400>  3
ctaatacgac tcactatagg gagaacgtgt gctcttccga tctttttttt tttttttttt        60

tttttttv                                                                 68


<210>  4
<211>  25
<212>  DNA
<213>  Artificial Sequence

<220>
<223>  DNA probe


<220>
<221>  misc_feature
<222>  (20)..(25)
<223>  n is a, c, g, or t

<400>  4
cacgacgctc ttccgatctn nnnnn                                              25


<210>  5
<211>  43
<212>  DNA
<213>  Artificial Sequence

<220>
<223>  DNA probe

<400>  5
ctaatacgac tcactatagg gagatttttt tttttttttt ttv                          43


<210>  6
<211>  25
<212>  DNA
<213>  Artificial Sequence

<220>
<223>  DNA probe


<220>
<221>  misc_feature
<222>  (20)..(25)
<223>  n is a, c, g, or t

<400>  6
cacgacgctc ttccgatctn nnnnn                                              25


<210>  7
<211>  63
<212>  DNA
<213>  Artificial Sequence

<220>
<223>  DNA probe

<400>  7
ctaatacgac tcactatagg gagaacgtgt gctcttccga tctgtcattc tcctaagaag        60

cta                                                                      63


<210>  8
<211>  25
<212>  DNA
<213>  Artificial Sequence

<220>
<223>  DNA probe


<220>
<221>  misc_feature
<222>  (20)..(25)
<223>  n is a, c, g, or t

<400>  8
cacgacgctc ttccgatctn nnnnn                                          25

<210>  9
<211>  25
<212>  DNA
<213>  Artificial Sequence

<220>
<223>  DNA probe

<220>
<221>  misc_feature
<222>  (20)..(25)
<223>  n is a, c, g, or t

<400>  9
cacgacgctc ttccgatctn nnnnn                                          25

## Description

### Field of the invention

The present invention relates to the field of *in vitro* transcription to generate a plurality of nucleic acids corresponding to a template nucleic acid.

### Background of the invention

Rapid progress in the development of ribonucleic acid (RNA) sequencing, RNA-seq, technologies in recent years redefined the scope and scale of analyses in biology. Next generation sequencing (NGS)-based technologies for genomics, transcriptomics and epigenomics are now increasingly focusing on the comprehensive characterization of individual cells to measure both the variance of individual cells and the averaged expression values of all cells after pooling (Shapiro et al., Nat. Rev. Genet. 2013; 14: 618-630). The transcriptome-wide RNA sequencing at the level of a single cell was pioneered by using linear amplification by *in vitro* transcription (IVT) and exponential amplification by polymerase chain reaction (PCR). The method was initially applied to commercially available deoxyribonucleic acid (DNA) mi-croarray chips, whereas the first report of single cell transcriptome analysis based on an NGS platform was published in 2009 by Tang et al. (Nat. Methods 2009; 6: 377-382).

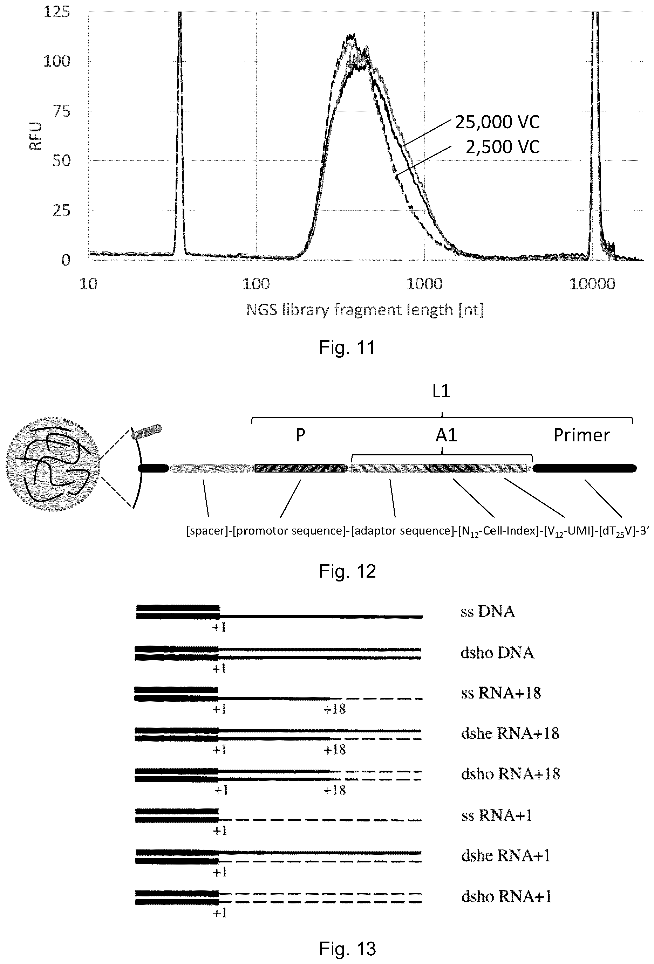

Linear RNA amplification is an isothermal nucleic acid amplification, also known as *in vitro* RNA-transcription-mediated amplification and is also referred to as amplified RNA (aRNA). aRNA is conventionally synthesized using the components of bacteriophages. The most frequently used systems originate from T3, T7 and SP6 bacteriophages. The DNA-dependent RNA polymerases (RNAP) exhibit strict specificity for specific promoter sequences. RNAPs catalyze *in vitro* RNA synthesis either on single-stranded DNA, double-stranded DNA, or single-stranded RNA starting at a cognate double-stranded promoter (Fig.13; Arnaud-Barbe et al., Nucleic Acids Res. 1998;26(15):3550-4). The basic strategy for aRNA generation is to place the promoter sequence upstream of any sequences of interest which can be either site, 3' or 5', of the original RNA template.

*IVT promoter sequence at the 3'position* Van Gelder et al. (Proc. Nat. Acad. Sci. USA 1990; 87: 1663-1667) used synthetic oligo(dT)-T7pr oligonucleotides that contained both a poly(dT) sequence and a phage T7 RNA polymerase promoter sequence to prime synthesis of complementary DNA (cDNA) by reverse transcription. Here, the poly(dT)-stretch of the primer selects for poly(A)-tails of mRNA species while the T7 promoter region initiates later the binding of T7 RNA polymerase for synthesizing RNA copies of the cDNA template. However, in this method the required double-stranded promoter sequence is generated only after second-strand cDNA synthesis which requires a second priming step. The IVT is using the second-strand cDNA as template and produces aRNA in antisense orientation (reverse complements). Examples in the literature can be found for IVT's from RNA templates (Nacheva et al. Eur J Biochem. 2003, 270(7):1458-65) and from single stranded RNA templates with a double stranded promoter (Arnaud-Barbe et al. Nucleic Acids Res. 1998, 26(15):3550-4). In direct comparison the most efficient IVT's result in the highest yields of a RNA were achieved from a template which consist of a double stranded DNA promoter followed by an RNA region in which the transition from DNA to RNA occurs 18 bases downstream from the promoter sequence.

*5'position of the IVT promoter sequence* Alternatively, the T7 promoter sequence can be introduced via template-switch (TS) oligonucleotides. Here, deoxycytidine addition through the terminal transferase activity of the reverse transcriptase enables base pairing with 3'nG-containing TS-oligonucleotides which serve as new template for continuing the elongation up to the 5' end of the TS-oligonucleotide. By these means a double-stranded promoter sequence is introduced at the 5' end of mRNA. A subsequent IVT synthesis copies of the RNA with the identical orientation.

In 2012 the aRNA method was modified to allow multiplexing of samples by using primers that contain unique cellular barcodes and Illumina platform compatible sequencing adaptor between the anchored poly(dT) and the T7 promoter sequences (Hashimshony et al., Cell Reports 2012; 2:666-673). The latter method was named CEL-seq and enabled many cells to be barcoded, linearly amplified and sequenced.

Despite wide adoption of these protocols, most are very inefficient, converting only very few percent of the RNA input molecules into libraries for sequencing. All, RNA purification, reverse transcription and second-strand DNA synthesis impose consecutive bottlenecks for converting RNA into double-stranded DNA copies. All sequences which are not captured by these initial reaction steps are lost for any subsequent amplification steps and finally the sequencing itself. Eliminating such barriers is important for low input material, e.g., RNA from single cells, but also for RNA input from complex tissue samples. One important aim of deep sequencing is to identify all RNA sequences without fail as rare transcripts often harbor clues to understand regulation, malfunction, and the development of diseases. Current approaches of single cell sequencing rely on labeling and sequencing RNA of large numbers of cells, sequencing with shallow read depth due to low conversion efficiencies, clustering of cells based on the high abundant marker transcripts, combining the reads of the cluster to obtain an averaged deep sequencing result of the cell clusters. Such methods depend on cell differentiation which are based on a small amount of high abundant transcripts only. High resolution in terms of gene expression levels is impossible.

Therefore, a method is needed which overcomes limitations in labelling and amplifying RNA molecules. Because each processing step of converting RNA into libraries for sequencing imposes efficiency bottlenecks, a method of amplifying RNA sequences starting from the RNA input itself is needed.

### Summary of the invention

The present invention provides a method for generating transcribed nucleic acids, comprising the steps of a) providing a nucleic acid template, b) hybridizing an oligonucleotide probe to the nucleic acid template, wherein said oligonucleotide probe comprises a complementary part that hybridizes to the nucleic acid template and a non-complementary part in 5' direction of the complementary part that does not hybridize to the nucleic acid template and comprises a sequence of a transcription promoter, c) hydrolysing a 3' part of the nucleic acid template that is in 3' direction to a part of the nucleic acid template that hybridizes to the oligonucleotide probe in step b) and wherein said 3' part is not hybridized to the oligonucleotide probe, d) extending the nucleic acid template with nucleic acids complementary to the non-complementary part of the oligonucleotide probe, thereby generating a duplex of the sequence of the transcription promoter in sequence with the nucleic acid template, e) transcribing the nucleic acid template with a transcriptase that binds the duplex of the sequence of the transcription promoter, thereby generating the transcribed nucleic acids.

In a further aspect the invention provides a set of a plurality of oligonucleotide probes suitable for the method of the invention, wherein said oligonucleotide probes each comprises a complementary sequence to a template sequence of choice, a transcription promoter sequence and an identifier sequence of at least 4 nucleotides in length.

In a related aspect, the invention provides a kit suitable for performing a method of the invention, comprising oligonucleotide probes comprising a transcription promoter sequence, a 3'->5' exonuclease, a DNA or RNA polymerase, and a transcriptase capable of initiating transcription at the transcription promoter sequence.

All aspects of the invention, the methods, sets and kits all relate to the invention and the specific embodiments described herein together, e.g. the methods can utilize the sets, their components, the kits or the components; the sets and kits can be suitable to perform any method of the invention and may comprise the components for said method.

### Brief description of the drawings

**Figure 1** Schematic reaction scheme for producing amplified antisense RNA (aRNA) from RNA by using an L1 oligonucleotide probe with the promotor sequence P.
**Figure 2** Bioanalyzer traces of 1 ng total RNA as input material and synthesized aRNA product using an L1 oligonucleotide probe with a poly(dT) complementary sequence.
**Figure 3** Schematic reaction scheme for producing amplified antisense RNA (aRNA) from RNA by using a L1 oligonucleotide probe with the promotor sequence P with an intermediate step of cDNA synthesis through reverse transcription.
**Figure 4** Bioanalyzer traces of 20 ng total RNA as input material and synthesized aRNA product using an L1 oligonucleotide probe with a poly(dT) complementary sequence an intermediate step of cDNA synthesis through reverse transcription.
**Figure 5** Schematic reaction scheme for producing aRNA and subsequently NGS libraries by using an L1 oligonucleotide probe with promotor region P and adaptor sequence A1 and a second primer with the adaptor sequence A2.
**Figure 6** Duplicates of Bioanalyzer traces of aRNA-3'seq NGS libraries generated from **A)** 10 pg total UHRR, **B)** 1 FACS sorted HEK293 cell, **C)** 100 pg total UHRR, and **D)** 10 FACS sorted HEK293 cells. The PCR cycles were applied to reach 2 nM indexed NGS libraries for sequencing.
**Figure 7** Comparison of aRNA-3'seq NGS reads mapping statistics for experiments starting with either 10 and 100 pg purified RNA (UHRR, or 1 and 10 FACS-sorted lysed HEK293 cells which contain RNA but also genomic DNA.
**Figure 8** Box plots of the number of identified genes (CPM>1) from 6 replicates of 10 pg universal human reference RNA (ThermoFisher Scientific, QS0639) using method 1 (M1, NEBNext^{®} Single Cell/Low Input RNA Library Prep Kit for Illumina^{®}, NEB), and the published data from method 2 (M2, SMART-seq2, Takara Bio, www.takarabio.com/learning-centers/next-generation-sequencing/technical-notes/single-cell-rna-and-dna-seq/highest-sensitivity-for-single-cell-mrna-seq), and aRNA-3'seq show for the latter higher detection rates and lower variance.
**Figure 9** Correlation plot of the mean gene expression values between 2× 4 HEK293 cells.
**Figure 10** Normalized gene body coverage plots using UHRR as starting material for aRNA-3'seq and aRNA-WTS NGS library preparations and mapping reads across all annotated human genes. Calculated by the RSeQC-2.6.4 package (Wang, Wang and Li, 2012).
**Figure 11** Bioanalyzer traces of targeted aRNA-seq NGS libraries which used L1 oligonucleotides containing complementary sequences to SARS-CoV-2, and L2 oligonucleotides with random sequences. The input material was SARS-CoV-2 reference RNA with nominal 2,500 and 25,000 virus copies (VC) per reaction.
**Figure 12** Schematic structure of beads which are covalently modified with L1 oligonucleotides for carrying out the aRNA workflow and NGS library generation at solid bead surfaces.
**Figure 13** Schematic overview about templates for *in vitro* transcription (IVT) from Arnaud-Barbe et al. (Nucleic Acids Res. 1998, 26(15):3550-4). The top strand is the non-template strand and the bottom strand is the template strand. Thick lines represent the consensus double stranded DNA promoter region; fine lines are DNA regions; dashed lines are RNA regions; +1 corresponds to the start site of transcription; +18 corresponds to the 18 base downstream from the start site; ss DNA, single stranded DNA template; dsho DNA, double stranded homoduplex DNA template; ss RNA+18, single stranded RNA+18 template; dshe RNA+18, double stranded heteroduplex RNA+18 template; dsho RNA+18, double stranded homoduplex RNA+18 template; ss RNA+1, single stranded RNA+1 template; dshe RNA+1, double stranded heteroduplex RNA+1 template; dsho RNA+1, double stranded homoduplex RNA+1 template.

### Detailed description of the invention

A method of generating transcribed nucleic acids. The inventive method allows for converting nucleic acid sequences, such as RNA sequences, into next-generation sequencing libraries.

In step a), a nucleic acid template (also referred to as just "template") is provided. The nucleic acid template is named so for containing the template sequence for transcription, which is performed within the inventive method in order to amplify and generate nucleic acid molecules (transcripts) comprising a nucleic acid sequence of the template. The nucleic acid template may be RNA or DNA. The inventive method is particularly suitable to analyse RNA templates, such as messenger RNA (mRNA), a non-coding RNA (ncRNA), a ribosomal RNA (rRNA), a microRNA (miRNA), which are therefore preferred nucleic acid templates. Any one of these or a combination thereof may be provided as nucleic acid template. Mixed type nucleic acid templates are possible, such as comprising both DNA and RNA. Preferably the nucleic acid template comprises or consists of RNA.

As used herein, the words "comprising" (and any form of comprising, such as "comprise" and "comprises"), "having" (and any form of having, such as "have" and "has"), "including" (and any form of including, such as "includes" and "include") or "containing" (and any form of containing, such as "contains" and "contain") are inclusive or open-ended and do not exclude additional, unrecited elements or method steps. The "comprising" expressions when used on an element in combination with a numerical range of a certain value of that element means that the element is limited to that range and "comprising" relates to the optional presence of other elements. E.g. the element with a range may be subject to an implicit proviso excluding the presence of that element in an amount outside of that range. As used herein, the phrase "consisting essentially of" requires the specified integer(s) or steps as well as those that do not materially affect the character or function of the claimed invention. As used herein, the closed term "consisting" is used to indicate the presence of the recited elements only.

The template can be provided in a mixture with other nucleic acid molecules, even with other nucleic acid types (e.g. of RNA or DNA) than the template.

Preferably the nucleic acid template is in a length of 20 to 100,0000 nucleotides, preferably 50 to 20,000 nucleotides.

The provided template may be purified or isolated from a cell, or provided non-purified, e.g. as a cell lysate. In case of the use of cell lysates, nucleases that digest the template are preferably inactivated, e.g. by denaturation such as by increasing the temperature, or by digestion, e.g. enzymatic cleavage of the nucleases. In case the template is RNA, the nuclease to be inactivated are RNases in case the template is DNA the nucleases to be inactivated are DNases. Preferably this step is a treatment with a proteinase, e.g. a proteinase that inactivates RNases and/or DNases.

Then in step b) of the inventive method an oligonucleotide probe is hybridized to the nucleic acid template. The oligonucleotide probe shall comprise a complementary part that hybridizes to the nucleic acid template and a non-complementary part in 5' direction of the complementary part that does not hybridize to the nucleic acid template. The non-complementary part comprises a sequence of a transcription promoter.

Oligo- and polynucleotide molecules have a directionality according to the 5' and 3' ends of the molecule. A "5' direction", also referred as "upstream" or "3'->5' direction", refers to a direction towards the 5' end. A "3' direction", also referred as "downstream" or "5'->3' direction", refers to a direction towards the 3' end. This directionality is given with regard to a molecule or strand that is described - this may or may not be a coding strand. A complementary strand being in a hybrid to the described oligo- and polynucleotide molecule has the opposite directionality.

"Complementary part" and "non-complementary part" refers to the complementary between the nucleic acids' nucleotides that form the hybrid or do not form the hybrid, respectively, in step a). Complementarities may change by changing nucleotides in later method steps. Preferably the complementary part is 10 to 100 nucleotides in length, preferably 12 to 50 nucleotides in length.

Hybridizing the oligonucleotide probe to the nucleotide template may form nucleotide hybrids with the template. The oligonucleotide probe may be extendable by a polymerase (as a primer) or not. Preferably the oligonucleotide probe is a DNA molecule, in particular comprising DNA nucleotides in the complementary part that hybridizes to the primer. The oligonucleotide probe may also comprise modified nucleic acids like LNA nucleotides, 2'-fluoro nucleotides, or 2'-O-methyl nucleotides. If the template is RNA, as in preferred cases, then the hybrid may be an RNA-DNA hybrid.

Through the oligonucleotide probe the method introduces a transcription promoter sequence suitable for in vitro transcription (IVT). This promoter may be in 5' direction to the complementary part. This transcription promoter sequence should be a single strand at step d) during the method so that a double strand of the transcription promoter is generated in continuity with the template, e.g. by extending the template with a polymerase in step d) or by e.g. ligating a complementary oligonucleotide in step d). A complementary oligonucleotide may also be present as a double strand in the oligonucleotide probe, wherein said complementary oligonucleotide that is hybridized to the transcription promoter is then ligated to the template. Preferably, the transcription promoter is maintained as single strand also in step b) and/or step c).

The transcription promoter sequence P can be any promoter sequence capable of initiating transcription in an *in vitro* setup. The promoter may be a T7, T3 or SP6 or any other promoter for which transcriptases, preferably RNA polymerases, are available. The promoters are used together with corresponding transcriptases in step e), e.g. T7, T3 and SP6 RNA polymerases. In preferred embodiments, the promoter is a T7 promoter. The orientation of the promoter sequence in the primer is such to initiate transcription towards the 5' end of the nucleic acid template.

The inventive method further comprises c) hydrolysing a 3' part of the nucleic acid template that is in 3' direction to a part of the nucleic acid template that hybridizes to the oligonucleotide probe in step b). Said 3' part of the nucleic acid template should not be hybridized to the oligonucleotide probe as the 3' part, if aligned to the oligonucleotide probe without deletions or insertions, would come to rest with the oligonucleotide probe's non-complementary part.

This 3' part of the nucleic acid template is also referred to a single stranded 3' overhang. The hydrolysis in step c) is preferably single strand specific in all embodiments of the invention. Preferably it is a step-wise hydrolysis, one nucleotide at a time from the 3' end in 5' direction until a duplex is reached, i.e. no further single strand is available for single strand specific hydrolysis. Exonucleases can perform such a reaction.

Hydrolysing this 3' overhang may be digestion, in particular enzymatic digestion. It should be specific to such a single stranded 3' overhang thereby maintaining the template being hybridized to the oligonucleotide probe. A preferred example of such a hydrolysis is with an exonuclease, preferably a single-stranded specific exonuclease that catalyses the removal of nucleotides in 3'->5' direction. Preferably the single-stranded specific exonuclease is a single-stranded RNA specific exonuclease in case the template, at least in the 3' overhang, is RNA.

The inventive method then continues with step d) extending the nucleic acid template with nucleic acids (nucleotides) complementary to the non-complementary part of the oligonucleotide probe, thereby generating a duplex of the sequence of the transcription promoter in sequence with the nucleic acid template. By extending the nucleic acid template (e.g. from a sample) on its 3' end (the remaining end up to which hydrolysis was done in step c)) in a "template-specific manner" (with the "template" for the elongation being exceptionally the oligonucleotide probe in this case) a longer complementary region in a duplex is generated between the nucleic acid template and the oligonucleotide probe that extends to and includes the transcription promoter, which originally in step b) was in a non-complementary part.

Such an extension reaction follows the principles of any primer-based extension in a 5'->3' direction, such as by using a polymerase. Alternatively, a ligation with one or more nucleic acids that are complementary to the oligonucleotide probe in 5' direction of the oligonucleotide probe is possible to generate the duplex with this 5' part of the oligonucleotide probe and - after ligation to the nucleic acid template - and the nucleic acid template (here a 3' part being in 3' direction with the part that was hybridized with the oligonucleotide probe in step b) .

The extension is preferably using DNA nucleotides. E.g. if the nucleic acid template is RNA then a mixed RNA-DNA molecule/strand is generated in this case.

The extension of the template is preferably through nucleotide polymerization, e.g. by using a polymerase, in particular preferred by using a DNA polymerase. By these means the introduced single strand promotor sequence (of the oligonucleotide probe) becomes a double strand, preferably a DNA double strand (when the oligonucleotide probe is also DNA).

The inventive method continues with e) transcribing the nucleic acid template with a transcriptase that binds the duplex of the sequence of the transcription promoter. This step of generating the transcribed nucleic acids, in particular in several copies - depending on the time allowed and on the amount of nucleotides provided for the transcribing reaction -, is by using a transcriptase enzyme. The generated transcribed nucleic acids are also referred as "transcripts". The reaction is preferably the linear *in vitro* transcription (IVT) which uses the nucleic acid template molecule (as modified in the preceding steps) as template. Transcription preferably generates RNA transcripts, which are also referred to as amplified antisense RNA or "aRNA", since they are antisense to the nucleic acid template when being transcribed from the nucleic acid template directly and not from a cDNA thereof.

The resulting transcripts (e.g. aRNA) can be used for downstream analyses, e.g., for preparing tagged libraries for sequencing, in particular preferred for direct RNA sequencing by third generation sequencing approaches like nanopore sequencing but also for next-generation "short read" sequencing. At this stage, fragmenting and adaptor tagging reactions do not impose limiting steps anymore because all transcripts are present already in numerous copies.

An advantage of the inventive method is that a nucleic acid template analysis of a sample has an increased efficiency, in particular generating transcripts from a higher percentage of the template molecules (see problem description discussed in background section). Transcriptome or gene expression analyses using the inventive method possess much improved sensitivity. The method can also circumvent problems with amplification by polymerase chain reactions (PCR) which tends to interfere with unbiased abundance determination when PCR efficiencies vary for different sequence fragments. The inventive method allows to convert close to all, but more than 75%, nucleic acid template molecules into transcripts, and linked thereto into libraries for sequencing. The method can start with tiny total nucleic acid template amounts, such as less than 10,000 molecules but also more, which are e.g. contained as messenger RNA (mRNA) in single cells or 10 pg purified total RNA and below or more. Preferably the nucleic acid template is present in amounts of 1,000 to 1,000,000,000 molecules, preferably 10,000 to 100,000,000 molecules, more preferred 50,000 to 10,000,000 molecules, in particular preferred 100,000 to 1,000,000 molecules.

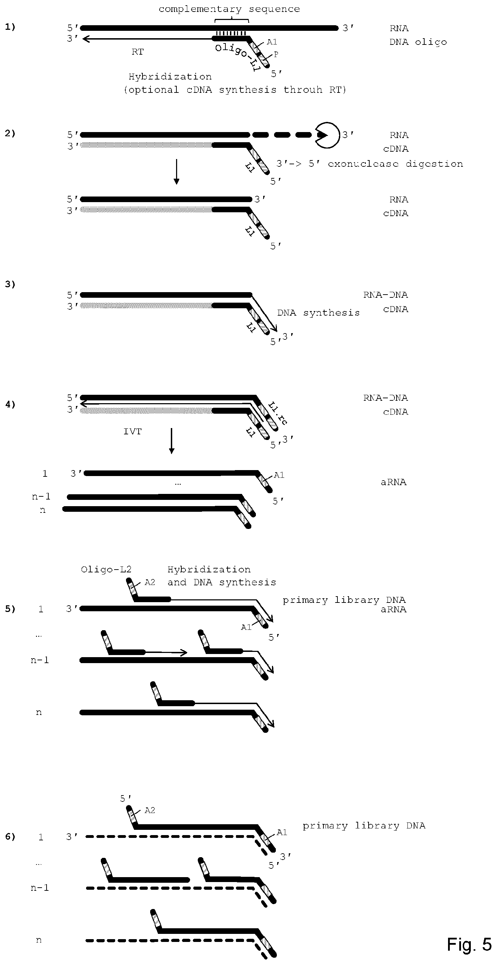

In a preferment, the inventive method further comprises the step of extending the oligonucleotide probe when hybridized to the nucleic acid template from the complementary part. In this case the oligonucleotide probe acts as a primer and is extended in the probe's 3' direction by adding nucleotides in a template-dependent manner (with the nucleic acid template being the template for this reaction). Preferably for this reaction the complementary part comprises an end nucleotide of the oligonucleotide probe molecule, i.e. an end that can be extended, preferably a 3'-end. To be extendible an accessible 3' OH should be present. This extension is preferably a polymerase reaction. In particular preferred, in case with the template being RNA (at least on the part in the extension direction, i.e. 5' from the template-probe hybridized part with reference to the template), the extension is preferably a reverse transcription (RT) using a reverse transcriptase (see Fig. 5, step 1)).

This extending the oligonucleotide probe is preferably performed after step b), before or after step c), before or after step d) but before step e). In particular preferred it is after step b) and before step c).

The step of extending the oligonucleotide probe to generate a double strand of the region in 3' direction of the complementary part with respect to the oligonucleotide probe increases stability of the molecules and/or opens and/or removes secondary structure of template nucleic acids in particular that of RNA and thus increases the efficiency of the method. To increase the benefit from this improvement, the step is preferably performed early on, e.g. before step c).

In preferred embodiments, the non-complementary part of the oligonucleotide probe as defined in step a) (5' to the complementary part that hybridizes with the nucleic acid template in step a)), comprises between the complementary part and the sequence of the transcription promoter an identifier sequence and/or a first adaptor sequence.

Such an identifier sequence can identify a sample, cell or nucleic acid template as provided in step a) in one container. Identifier sequences allow multiplex reactions later on, when nucleic acid templates and/or transcripts are later pooled together for later method steps. Even in a mixture of several nucleic acid templates and/or transcripts and/or their amplificated copies can be identified for belonging to a certain sample, cell or nucleic acid template according to the identifier sequence which is different from sample to sample or cell to cell or nucleic acid template to nucleic acid template. Of course, any such identifiers can be combined (see e.g. Fig. 12 with a nucleic acid template specific identifier (UMI) and a cell specific identifier ("Cell Index"). Such a sample specific identifier sequence is also referred to as a "sample barcode". Other identifier sequences can be sequences which identify multiple copies of identical sequences like transcript copies in one sample or from one cell. Such identifier sequences are unique molecular identifiers (UMI). Sample identifiers may be used on oligonucleotide probes, wherein all oligonucleotide probes used on a particular sample have the same identifier, but different identifier when applied to a different sample; cell identifiers may be used on oligonucleotide probes, wherein all oligonucleotide probes used on a particular cell have the same identifier, but different identifier when applied to a different cell. Samples or cells may be isolated from other samples or cells, respectively, for the treatment with the oligonucleotide probe. Nucleic acid template specific identifiers (UMI) are usually used with each oligonucleotide probe comprising a different identifier. No isolation of individual molecules is needed in this case but of course still possible. Sample or cell or nucleic acid template specific identifier sequences are preferably sequences of 4 to 16, preferably 6 to 12, nucleotides in length. Different identifier sequences that identify different samples of nucleic acid templates, that identify different cells or that identify different nucleic acid templates, respectively, differ to each other, preferably by a Hamming distance of at least 1, preferably 2 or more, preferably 3 or more, or preferably a Levenshtein distance of at least 1, preferably 2 or more, preferably 3 or more. The nucleic acid template specific identifiers (UMI) are preferably different for all molecules of identical sequence, which means that the number of UMI's exceeds the number of the highest abundant transcripts, preferably exceeds this abundance by a factor of 10 when the UMI's are made by random sequences. The transcripts that are obtained by the inventive method, which are copies originating from one specific template, can then be traced by the UMI to originating from one template molecule. Alternatively or in combination thereto, e.g. by using two identifier sequences, a sample or cell may be identified with one identifier sequence while the UMI identifies the template molecule. The identifier sequences are preferably in 3' direction to the promoter with respect to the oligonucleotide probe to be copied by the IVT reaction.

In preferred embodiments, such adaptor is compatible to sequencing adaptors for nanopore sequencing. For example, the adaptor may comprise a sequence for nanopore motor protein binding, e.g. a Y-adaptor.

An adaptor sequence may or may not be the same for all samples/nucleic acid templates. Preferably it is the same for all samples/nucleic acid templates. The adaptor can be used to bind primers or probes to a nucleic acid with a sequence comprising the adaptor sequence or its complementary sequence or to bind the nucleic acid with a sequence comprising the adaptor sequence or its complementary sequence to nucleic acids that hybridize to the adaptor sequence or its complementary sequence. The nucleic acid with a sequence comprising the adaptor sequence may be a transcript or a further amplification product thereof. The adaptor thus enables further handling or further amplification of any nucleic acid that contains it or its complementary sequence. An adaptor sequence is preferably 4 to 30 nucleotides in length, particularly preferred 6 to 25 nucleotides in length, or even more preferred 8 to 20 nucleotides in length.

The adaptor sequence is preferably in 3' direction to the promoter with respect to the oligonucleotide probe.

In further preferred embodiments, the inventive method comprises hybridizing one or more secondary primers to the one or more transcribed nucleic acids and extending said secondary primers in a template-dependent manner. By binding a secondary primer to the transcripts and extending said primer with the transcript as template, nucleic acid molecules with complementary sequences to the transcript are obtained (see step 5 in figure 5; the secondary primer is referred to as "Oligo-L2" therein). Such secondary primers may bind to the transcript at any sequence part or on specific sequences that can be preselected based on the expected or known sequence of the transcript. Binding at any part of the transcript can e.g. be facilitated by random primers, e.g. a mixture of primers with various hybridizing sequences, such as random oligomer, e.g. random hexamer, primers. The secondary primer is preferably hybridized to the transcript with a sequence of 5 to 30 nucleotides in length, preferably 6 to 20 nucleotides in length.

Preferably the secondary primer(s) comprise(s) a second adaptor sequence. The adaptor sequence is preferably the same for all secondary primers irrespective of the transcript-binding sequence. The adaptor can be used to bind primers or probes to a nucleic acid with a sequence comprising the adaptor sequence or its complementary sequence or to bind the nucleic acid with a sequence comprising the adaptor sequence or its complementary sequence to nucleic acids that hybridize to the adaptor sequence or its complementary sequence. The nucleic acid with a sequence comprising the second adaptor sequence may be a nucleic acid molecule as described above. The adaptor enables further handling or further amplification of any nucleic acid that contains it or its complementary sequence. A second adaptor sequence is preferably 4 to 30 nucleotides in length, particularly preferred 6 to 25 nucleotides in length, or even more preferred 8 to 20 nucleotides in length. The secondary primer together with the adaptor sequence on the oligonucleotide primer can in only one amplification step (1st strand synthesis) generate a complete fragment with two adaptors which can be used directly (i.e. without further amplification or PCR) in NGS runs. The adaptor of the secondary primer may optionally have an identifier sequence. The same as was described for the identifier sequence of the oligonucleotide probe applies to this optional identifier sequence of the secondary primer.

In particular preferred is the combination of an adaptor on the non-complementary part (as present in step b)) and an adaptor on the secondary primer. This allows the generation of nucleic acid molecules that have adaptors or their complementary sequence on or near both ends, i.e. flanking the template sequence of interest. This allows the selection and amplification in one or more cycles of PCR or binding on a sequencing solid phase by one or both adaptor sequences or the complementary sequences thereto.

The inventive method has the benefit of a very high sensitivity. This allows the analysis of nucleic acids from single cells or their organelles or compartments such as mitochondria (e.g. mtRNA) or exosomes. Preferably the nucleic acid template comprises or consists of RNA or DNA, preferably RNA, from organelles, cell sections, or cells, preferably from 1 to 1000 cells, organelles, cell sections or cells. Cell sections are for example cut dendrites in, e.g., spacial transcriptomic workflows. Such cell sections may comprise characteristic RNA as suitable template for the inventive method.

The nucleic acid templates may be present in a mixture of nucleic acids, including nucleic acids that may not be templates such as other types of nucleic acids (RNA or DNA) or comprising sequences to which the oligonucleotide probe will not bind. A further category of nucleic acids that are not templates can be nucleic acids that are removed or made inaccessible from further processing, e.g. from oligonucleotide probe hybridization in step b), hydrolysing the 3' part in step c) or extending the nucleic acid template in step d). In particular preferred embodiments, the nucleic acid template is in a pool of nucleic acids comprising DNA. The nucleic acid template is preferably RNA. Preferably the DNA is not hydrolysed in step c) while RNA is. For example, the RNA 3' part can be digested by an RNA specific exonuclease. Accordingly, such non-template nucleic acids, like DNA when the template is RNA, become excluded from any subsequent transcription in step e) by failing to be processed in steps b), c), d) or e). This has the benefit that in this case of RNA templates, genomic contamination (from DNA) of the product transcribed nucleic acids is reduced.

A further advantage of the inventive method is that it can be done in mixture of other nucleic acids. No purification of the reaction products of step a), b), c), d), and e) or any combination thereof, such as steps b) and c), or steps c) and d), or steps b)-d), in particular all of these steps, is needed.

For easy handling, the steps are done in one container, such as a flask, vial, bag, syringe, or a well, including a microwell on a well-plate, or any other holding means or suitable encapsulation. In preferred embodiments, the inventive method comprises providing the nucleic acid template in a container and performing steps b) to e) in said container. The template preferably remains in said container until step e) and/or is not removed from said container before step e). No washing or purification of the template may be needed before step b). Some steps can even be performed in conjunction, e.g. steps c) and d). Accordingly, an exonuclease suitable for step c) and a polymerase suitable for step d) can be combined in the reaction mixture. The polymerase will become active once the exonuclease has removed the 3' overhang of the template (blunted) to the complementary part of the oligonucleotide probe so that the polymerase can now be active and start extending the duplex of the template and the probe by extending the template in dependence of the (previously in step b)) non-complementary part of the oligonucleotide probe.

Preferably oligonucleotide probes are removed, e.g. by binding to a solid phase, before step e).

The inventive method can be performed in one single gradually increasing volume, by just adding further reagents to the reaction mixture to complement the enzymatic activities and reaction conditions.

When providing nucleic acids from cells, cell contents, such as enzymes, that would degrade the nucleic acid template or interfere with reactions of the subsequent reactions of the method, should be inactivated so that the nucleic acid template is not affected. The inventive method preferably comprises providing one or more cells, e.g. 1-1,000 cells, lysing cell material, inactivating enzymes, preferably by a proteinase, thereby proving nucleic acids of said cells as nucleic acid template according to step a). Preferably RNases are inactivated, in particular when the template comprises or consists of RNA; preferably DNases are inactivated, in particular when the template comprises or consists of DNA. An example proteinase is proteinase k. After preparing the nucleic acid templates the proteinase is preferably inactivated, e.g. heat inactivated. Subsequently, e.g. in steps b) to e), no heat inactivation is needed but can of course be optionally performed.

In a particular embodiment of the invention the depletion of ribosomal RNA (rRNA) can be avoided or enhanced through selective priming, which discriminates against or selects respectively, rRNA.

The invention further provides a set of a plurality of oligonucleotide probes suitable for the inventive method. In the set, said oligonucleotide probes each comprises a complementary sequence to a template sequence of choice, a transcription promoter sequence and an identifier sequence of at least 4 nucleotides in length. A complementary sequence to a template sequence of choice is e.g. a sequence of a poly(dT)-sequence of at least 6 consecutive T's. Such a poly(dT)-sequence is complementary to a poly(A)-sequence that is found on template, such as mRNA.

The plurality is preferably at least 2, 3, 4, 5, 6, 7, 8, 9, 10, 11, 12, 13,14, 15, 16, 17, 18, 19, 20 or more; such as 2 to 100,000,000. "Plurality of oligonucleotide probes" refers to various oligonucleotide probe molecules. These oligonucleotide probe molecules may or may not differ in their nucleotide sequence. Preferably 2, 3, 4, 5, 6, 7, 8, 9, 10, 11, 12, 13,14, 15, 16, 17, 18, 19, 20 or more different oligonucleotide probes are provided in said plurality, e.g. 2-1,000 different oligonucleotide probes. Differences are preferably in the identifier sequence. The identifier sequence may be as discussed above, such as being preferably 4 to 12 nucleotides in length. Different identifier sequences preferably differ to each other by a Hamming distance of at least, preferably 2 or more, preferably 3 or more, or a Levenshtein distance of at least, preferably 2 or more, preferably 3 or more. Preferably the identifier sequence is different for at least two oligonucleotide probes of the plurality. The identifier sequence may be a sample, cell or nucleic acid template specific identifier sequence as mentioned above. For nucleic acid template specific identifier sequences, preferably each oligonucleotide probe comprises a different identifier sequence that is different from identifier sequences of other oligonucleotide probes. For cell specific or sample specific identifier sequences, the set may comprise groups of oligonucleotide probes that within a group have the same identifier sequence that differs from identifier sequences of other groups of oligonucleotide probes. The set may comprise 2, 3, 4, 5, 6 or more such different groups. Nucleic acid template specific identifier sequences and cell and/or sample specific identifier sequences may be used simultaneously, e.g. with a probe of the set comprising two or more identifier sequences. Preferably the transcription promoter sequence is the same for the plurality of oligonucleotide probes.

In preferred embodiments, the transcription promoter sequence of the oligonucleotide probes of the set is single stranded. As discussed above, it may alternatively be double-stranded but it should be turned single-stranded before step d) so that extension of the nucleic acid template can occur. In preferred embodiments, the oligonucleotide probes of the set are single-stranded in their entire length.

The present invention further provides a kit suitable for performing the inventive methods. A provided kit comprises oligonucleotide probes comprising a transcription promoter sequence, a 3'->5' exonuclease, a DNA or RNA polymerase, and a transcriptase capable of initiating transcription at the transcription promoter sequence.

In preferred embodiments, the kit further comprises dNTPs, a cell lysis reagent, a proteinase, a reverse transcriptase, or any combination thereof. The kit may also provide the oligonucleotide probes of the set as described above. E.g. the transcription promoter sequence may be the promoter of the T7 RNA polymerase, the T3 RNA polymerase or the SP6 RNA polymerase.

The oligonucleotide probes are preferably 12 to 100 nucleotides in length. They may be as described above with regard to the inventive method.

The 3'->5' exonuclease shall be suitable for step c) of the inventive method. An example is an exoribonuclease.

The DNA or RNA polymerase shall be suitable for step d) of the inventive method, to extend the nucleic acid template to generate the double strand with the (previously in step b)) non-complementary part of the oligonucleotide probe.

The transcriptase shall be suitable for step e) of the inventive method and facilitate transcription in dependence on the presence of a functional transcription promoter sequence, i.e. in duplex as generated in step d).

Further optional components of the kit may be nucleotides, e.g. dNTPs and/or NTPs. Nucleotides should be suitable for the extension in step d) or the transcription in step e) to add nucleotides to a primer a probe or the template. Since nucleotides are usually available in a laboratory, they may not be provided with the inventive kit as they are abundantly available.

A cell lysis reagent may be present for step a) to provide the nucleic acid templates from cells. Again, as this compound is readily available elsewhere, it may not be provided with the kit, but preferably it is included for convenience.

A proteinase may be included in the kit to remove any RNases or DNases in a cell lysate. This component is also optional as it is available elsewhere.

A reverse transcriptase may be included for the optional step of extending the oligonucleotide probe in 3' direction in dependence of the nucleic acid template. As noted above, this can increase stability of the template and increase sensitivity of the inventive method.

The set and/or kit may further comprise instructions to perform the inventive method.

The set/or kit may comprise any one selected from stabilizers, carriers, buffers, solvents, containers, salts, tonicity-adjusting agents, fillers, antimicrobial agents, isotonic agents, antioxidants, and other conventional composition agents or combinations thereof.

The kit comprises such containers, wherein the containers are packaged together, e.g. in a packaging envelope such as a box or bag.

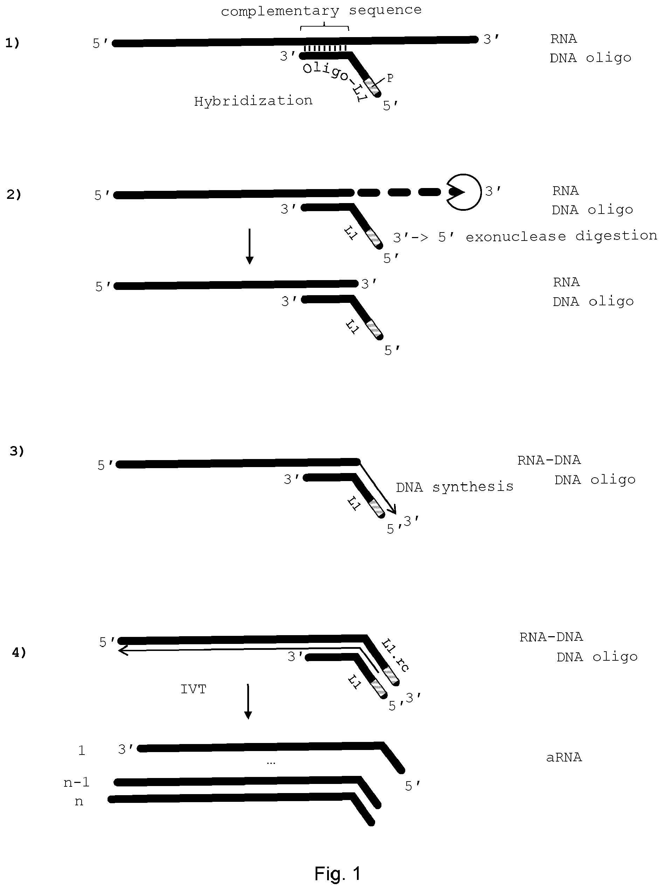

In particular preferred embodiments, the present invention enables the synthesis of amplified antisense RNA (aRNA) directly from nucleic acid templates, including from RNA templates. Provided are methods and kits suitable for transcriptome profiling and the labelling and amplifying of template molecules from ultralow input material and single cells. Particular preferred embodiments are described in the following in reference to **Figure 1** in more detail:
(1) Hybridization (step b): of an oligonucleotide probe, labelled as "oligo L1", which contains a transcription promotor sequence P and a complementary sequence to a nucleic acid template, here an RNA sequence,
(2) 3'->5' exonuclease digestion (step c): enzymatic hydrolysis of the nonhybridized 3'-overhang in template-oligonucleotide probe hybrid through an exonuclease, e.g. an RNA exonuclease for RNA templates,
(3) DNA synthesis (step d): elongation of the template through a DNA-polymerase using the protruding 5'-end of oligo L1 as template generating a double-stranded promotor region, and
(4) IVT (step e): linear amplification by IVT starting at the double-stranded promotor region using the "nucleic acid template" as template to synthesize multiple aRNA copies.

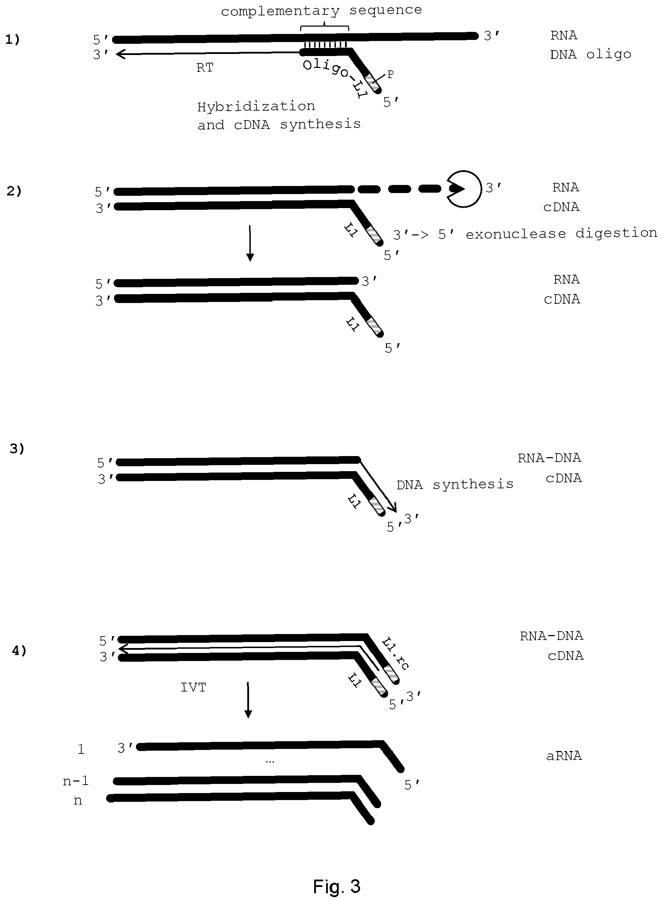

The reaction scheme is shown in **Figure 1** and **Figure 3****,** which contains an additional synthesis of complementary DNA (cDNA) to stabilize the RNA template through an RNA-DNA duplex, when the nucleic acid template is RNA; or DNA-DNA duplex when the nucleic acid template is DNA. Especially long RNA single strands may have reduced stability in elevated temperatures, e.g. 50°C.

In preferred embodiments of the invention, at the beginning an RNA sample is provided as nucleic acid template. The RNA is preferably a messenger RNA (mRNA), a non-coding RNA (ncRNA), a ribosomal RNA (rRNA), a microRNA (miRNA), or a mixture of different precursor and processed matured RNA which is present in purified RNA or in total RNA of cell lysates. The RNA may be of any length, but preferably is in the range of 20 to 10,000 nucleotides. The original RNA provides the template for synthesizing many copies in antisense orientation.

In step 1) / step b), the oligonucleotide probe, which includes one complementary sequence to the nucleic acid template, is added and annealed to the nucleic acid template by reducing the temperature or changing compounds, or their concentrations, in the buffer solution. The stabilized Watson-Crick base pairing hybridization produces a template-probe duplex, in preferred embodiments with the template being RNA and the probe being DNA with a short RNA-DNA double-strand. The probe contains a promotor sequence P which does not anneal to the template.

The complementary sequences of annealing regions are preferably optimized for high specificity. The complementary sequence can be, e.g., a poly(dT)V-3' sequence to bind to polyA-tails of messenger RNA (mRNA), a specific conserved sequence to bind to a targeted group of transcripts, or random sequences. To target the polyA-tails of mRNA is preferably done with oligo-dT8 to oligo-dT30 sequences. Preferable are oligo-dT15 to oligo-dT25 which decreases priming events elsewhere (internal priming to transcripts elsewhere as to the poly(A)-tail). An optional 3' anchor sequence of 3'V or alternatively 3'NV directs the hybridization to the 5'start of polyA-tails.

The complementary sequence can be a highly selective sequence, which specifically hybridizes to one or very few target sequences. Such complementary sequence contains preferably between 18 and 45 nucleotides. The length is preferably at least 8 nucleotides to stabilize the hybrid binding and allow later to be recognized by a polymerase in step c).

In one embodiment of the present invention, the template-probe duplex (preferably an RNA-DNA duplex) is stabilized through extending of the oligonucleotide probe, e.g. by reverse transcription (RNA-DNA) or a polymerase (DNA-DNA). The required reaction mixture includes a reverse transcriptase (or polymerase), a suitable buffer, dNTPs and optional RNase inhibitor(s). In preferred embodiments, the reverse transcription reaction is conducted in the presence of an additive(s), such as PEG, preferably PEG-8000, or albumin, preferably bovine serum albumin (BSA), to increase the yield of cDNA. In addition, or alternatively to PEG, other additives that may be added in the cDNA synthesis of the present invention include, but are not limited to, dimethyl sulfoxide (DMSO), trehalose, glucose and glycerol. In RT embodiments, the desired reverse transcriptase activity may be provided by any suitable enzyme that is MMLV-related, including but not limited to Superscript I, II, III or IV, Maxima H, RevertAid, SMARTScribe, EnzScript, ProtoScript II, GoScript or RNase H- mutants thereof. The reverse transcription reaction may be performed between 37 to 55°C in as little as 10 minutes or as much as 12 hours. In one embodiment, the reaction will be carried out between 10 to 30 min, or alternatively between 10 to 60 min, or alternatively between 10 to 120 min, or alternatively any reaction time can be used.

The following processing of RNA-DNA duplex includes two enzymatic steps which are carried out in one combined reaction setup.

In step 2) / step c), the template-probe duplex is treated with a single-strand (ssRNA or ssDNA) specific exoribonuclease(s) to remove the nonhybridized part of the template in 3' to 5' direction (blunting). In one embodiment, this can be the 3'-poly(A) tail from mRNA which exceeds the part of the hybridized RNA-DNA double-strand. In the digestion step, all ssRNA specific exoribonucleases such as RNase R, RNase T and RNase D can be used in case of the template 3' overhang being RNA. Exoribonucleases digest essentially all linear single-stranded RNA 3' overhangs but do not digest double-stranded RNA, or specifically double-stranded RNA-DNA duplexes. For single strand DNA duplexes DNA 3'->5' exonucleases can be used.

In one particular embodiment the complementary sequence of the oligonucleotide probe is a poly(dT)-strand, which binds to poly(A)-tails of mRNA. However, such poly(dT) sequences can also bind to T-rich sequences in RNA but also vast DNA background which is present for example in lysed single cells and all samples without extra RNA purification. In this 3'->5' exonuclease digestion step relative short single stranded RNA overhangs, in particular the remaining polyA-tails, are much more prone to an efficient complete digestion than any long fragments. The digestion of 3' located fragments of DNA chromosomes, which tend to be in addition partially rehybridized and double stranded, is hindered. In this case, the method can be used as a selective mRNA enrichment method because it discriminates any potential mispriming into DNA background, such as genomic DNA (gDNA).

Alternatively, the complementary sequence of the oligonucleotide probe can be any selective sequence complementary to any predefined target sequence on the template. Although mispriming of such sequences is in general less frequent than poly(dT)-priming, the priming to any present chromosomal DNA has also little consequences. The digestion of the 3'-located sections of DNA chromosomes downstream to a priming event which are partially rehybridized and double stranded is essentially impossible. Therefore, the method presents a highly efficient RNA enrichment method for RNA fragments 5' upstream of the targeted region.

In step 3 / step d), after digestion the remaining 3'-OH of the template group in the template-probe duplex is the starting point of an extension reaction, preferably a DNA polymerization with a DNA polymerase which uses the 5' overhang of the oligonucleotide probe for hybridizing the nucleotides of the extended template. The result is a duplex which now possesses a double-strand of the promotor sequence P of the oligonucleotide probe.

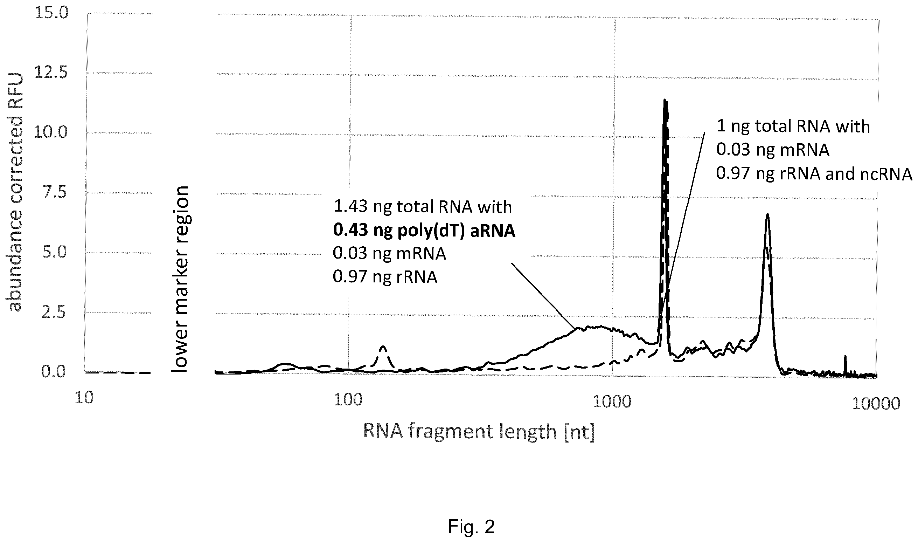

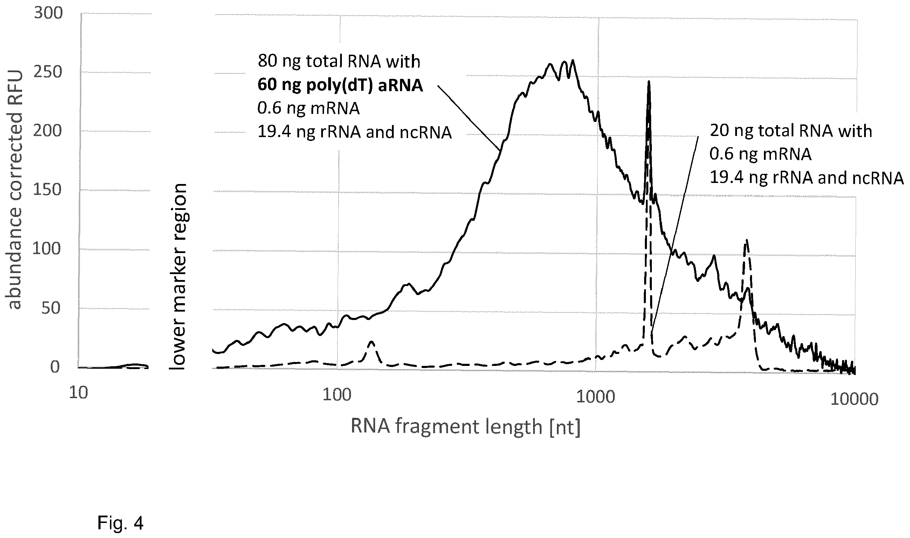

In step 4 / step e), the transcriptase (RNA polymerase) in the presence of all four (ribo)nucleotide-triphosphates and a compatible buffer binds to double stranded promoter sequence P and amplifies a single-stranded RNA using the extended nucleic acid template as template. Once an RNA polymerase left the region of stranded promoter sequence P, another RNA polymerase can bind and start a new synthesis. The continuous process results in a linear amplification of the template producing amplified antisense RNA (aRNA) with tens of copies in one process, typically 100 - 200 copies, but also 200 - 1,000 copies, or copies exceeding 1,000 are possible (**Figure 2** and **Figure 4**). The amplification is controlled by the amount of the reaction constituents, the reaction volume, the temperature and the reaction time.

The obtained aRNA can be used as input for any RNA sequencing library preparation.

Direct NGS Library preparation. In one particular embodiment the oligonucleotide probe contains an adapter sequence A1 between the promotor sequence P and the complementary sequence responsible for hybridizing to the template (**Figure 5**). The adapter sequence A1 can combine sequences of different functionality which are i) priming sites for subsequent amplifications with extended sequences, which are, e.g., required for different sequencing technologies to bind onto flow cells, ii) indices which can be either sample specific, random also known and used as unique molecular identifier (UMI), or both in succession, iii) complete adapters which can be sequenced directly without any amplification. Including all these embodiments, the oligonucleotide probe contains typically between 15 to 75 nucleotides.

Because the linear amplification in the present workflow generates vast amounts of 5' directed adapter A1 containing aR-NA, a subsequent first strand synthesis with primers containing complementary sequences for hybridization and the adaptor sequence A2 can be sufficient to use the single first strand directly for sequencing without the need for further PCR amplification. Alternatively, several indexed libraries can be pooled and concentrated to combine enough first strand libraries so that PCR amplification can be omitted. Omitting PCR amplification eliminates the possibility of producing PCR artefacts which are caused by different amplification efficiencies leading to changes in the measured abundance ratios.

The present invention is illustrated in the figures and following examples, without being limited to these embodiments of the invention.

### Examples

### Example 1: RNA amplification

The reaction scheme of producing aRNA from template RNA is shown in **Figure 1****.** The experiment was performed using universal human reference RNA (UHRR, Agilent Technologies, Catalog # 740000) using a probe L1 containing a T7 promoter sequence and a string of 20 deoxythymidylic acid residues followed by dV. In the present example the L1 oligonucleotide probe 5'-CTA ATA CGA CTC ACT ATA GGG AGA TTT TTT TTT TTT TTT TTT TTV-3' (Seq ID NO: 1) was used at a concentration of 500 pmol. Due to the end nucleotide the L1's are anchored at the 5'-start of poly(A)-tails of mRNA suppressing any priming downstream within the poly(A)-tails. In this particular example, the hybridization (step 1) used 1 ng of total UHRR, 0.5 mM free dNTPs and nuclease-free water. The RNA template and probe mix was heated at 72°C for 3 min, immediately transferred on ice and kept for 5 min on ice. The reaction solution containing the template-probe hybrid molecules was then pretreated with single-strand-specific exoribonuclease (step 2) and DNA polymerase for 30 min at room temperature (step 3). This template-probe end-repair reaction was performed in 50 µl with 20 mM Tris-acetate (pH 7.9 at 25°C), 10 mM Magnesium acetate, 50 mM Potassium acetate, 100 µ/ml BSA, 5 units of single-strand specific exoribonuclease and 3 units of DNA polymerase. Then, remaining oligonucleotide probes, dNTPs and small fragments were removed by solid phase reverse immobilization (SPRI) with magnetic purification beads (AMPure Beads, Agencourt). The linear amplification by *in vitro* transcription (step 4) was carried out on the template-probe-dsT7pr hybrids in the presence of 40 mM Tris-HCl (pH 7.9 at 25°C), 6 mM MgCl², 10 mM DTT, 10 mM NaCl, 2 mM Spermidine, 200 U T7 RNA polymerase, 3.5 mM rNTPs, and 10% DMSO at 37°C for 90 min. The resulting RNA transcripts were purified by SPRI. **Figure 2** shows corresponding traces of input total mRNA and the output of total RNA plus aR-NA. The difference between both traces shows 0.43 ng aRNA which is 14.3 times more than the mRNA of the input material.

### Example 2: RNA amplification with reverse transcription

The reaction scheme is shown in **Figure 3** and includes an added reverse transcription (RT). The RNA amplification experiment was made using 20 ng universal human reference RNA (UHRR, Agilent Technologies, Catalog # 740000). The reverse transcription was carried out in 10 µl in the presence of 0.5 mM free dNTPs, 500 pmol anchored L1 probe consisting of a T7 promoter sequence and a string of 25 deoxythymidylic acid residues followed by dV (5'-CTA ATA CGA CTC ACT ATA GGG AGA TTT TTT TTT TTT TTT TTT TTV-3' - Seq ID NO: 2), 50 mM Tris-HCl (pH 8.3 at 25°C), 75 mM KCl, 3 mM MgCl₂, 10 mM DTT and 200 U of reverse transcriptase for 15 min at 37°C. Without purification the reaction was then increased to 50 µl by adding 20 mM Tris-acetate (pH 7.9 at 25°C), 10 mM Magnesium acetate, 50 mM Potassium acetate, 100 µg/ml BSA, 5 units of single-strand specific exoribonuclease and 3 units of DNA polymerase. After the digestion and extension, the remaining oligonucleotide probes, dNTPs, and small fragments were removed by solid phase reverse immobilization (SPRI) with magnetic purification beads (AMPure Beads, Agencourt). The linear amplification by *in vitro* transcription was carried out on the remaining RNA-cDNA hybrids in the presence of 40 mM Tris-HCl (pH 7.9 at 25°C), 6 mM MgCl², 10 mM DTT, 10 mM NaCl, 2 mM Spermidine, 200 U T7 RNA polymerase, 3.5 mM rNTPs and 10% DMSO at 37°C for 12 hours. **Figure 4** shows corresponding traces of input total RNA and the output of total RNA plus aRNA. The difference between both traces shows ca. 60 ng aRNA which is approximately 100 times more than the mRNA of the input material. Due to the long reaction time the product trace contains some degraded rRNA which is seen by the lower 28s rRNA peak. However, the majority of the rRNA is still intact and influences only little the quantitative evaluation of the aRNA.

### Example 3: RNA amplification with NGS library generation

The reaction scheme is shown in **Figure 5****.** RNA experiments were performed using universal human reference RNA (UHRR, Agilent Technologies, Catalog # 740000) or FACS-sorted HEK293 cells for which 1 or 10 cells were directly sorted into wells containing 5 µL of lysis buffer (Lexogen GmbH). Cell lysis was carried out for 10 min at 50°C, and 10 min at 80°C. After cell lysis, or in the case of using directly 10 or 100 pg UHRR, which corresponds to the approximate total RNA amount of 1 and 10 cells, reverse transcription was carried out in the presence of 0.5 mM free dNTPs, 500 pmol anchored probe L1, consisting of a T7 promoter sequence, an adaptor sequence A1, and a string of 25 deoxythymidylic acid residues followed by dV, 5'-CT AAT ACG ACT CAC TAT AGG GAG AAC GTG TGC TCT TCC GAT CTT TTT TTT TTT TTT TTT TTT TTTTTV-3' - Seq ID NO: 3). RNA-cDNA hybrid molecules were directly treated with single-strand-specific exoribonuclease and DNA polymerase. The RNA-cDNA treatment was performed in 50 µl with 20 mM Tris-acetate (pH 7.9 at 25°C), 10 mM Magnesium acetate, 50 mM Potassium acetate, 100 µ/ml BSA, 5 units of single-strand specific exoribonuclease and 3 units of DNA polymerase. Remaining oligonucleotide probes, dNTPs and small fragments were removed by solid phase reverse immobilization (SPRI) with magnetic purification beads (AMPure Beads, Agencourt). The linear amplification by *in vitro* transcription was carried out on the remaining RNA-cDNA hybrids in the presence of 40 mM Tris-HCl (pH 7.9 at 25°C), 6 mM MgCl², 10 mM DTT, 10 mM NaCl, 2 mM Spermidine, 200 U T7 RNA polymerase, 3.5 mM rNTPs and 10% DMSO(at 37°C for 12 hours) .

The A1 containing aRNA was purified using 1.8x ratio of SPRI beads, performing the final elution in 10 µl of elution buffer (EB) solution (Lexogen GmbH). The purified A1 containing aRNA was then primed with a random hexamer primer, 5'-C ACG ACG CTC TTC CGA TCT NNN NNN-3' (Seq ID NO: 4), harboring an A2 adaptor sequence. Random priming onto the aRNA was achieved by incubating for 10 min at 25°C, 40 min at 37°C, 10 min at 42°C and 1 min at 25°C with reverse transcriptase being present in an RTM buffer (Lexogen GmbH). The reaction mix was SPRI purification and eluted in 20 µl EB (Lexogen GmbH). The primary libraries of ssDNA fragments contained now both adaptor sequences, A1 and A2 or complementary parts thereof, and were amplified in a PCR reaction using a DNA polymerase, PCR buffer and indexed primers (Lexogen GmbH). The PCR was performed by applying 98°C for 30s, followed by 9-13 cycles between 98°C for 10s, 68°C for 20s, and 72°C for 30s.

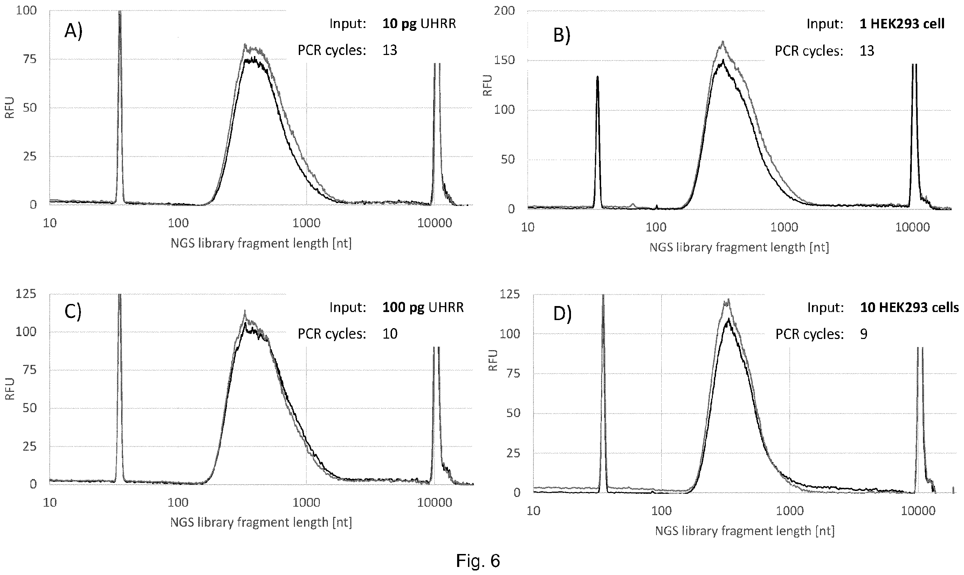

**Figure 6** shows Bioanalyzer traces of final NGS libraries. The initial amount of primary library can be calculated from the final yield and the applied number of PCR cycles using a measured PCR efficiency of 0.9. The initial amounts of primary libraries are very similar when comparing 10 pg UHRR and 1 HEK293 cell, or 100 pg and 10 HEK293 cells. It proves that the present aRNA protocol and NGS library preparation can start equally well with purified RNA and cell lysate which contains genomic DNA background.

Duplicates are highly consistent. Negative template controls (NTC) show flat lines at identical PCR cycles but produce some background when applying ca. 28-30 PCR cycles.

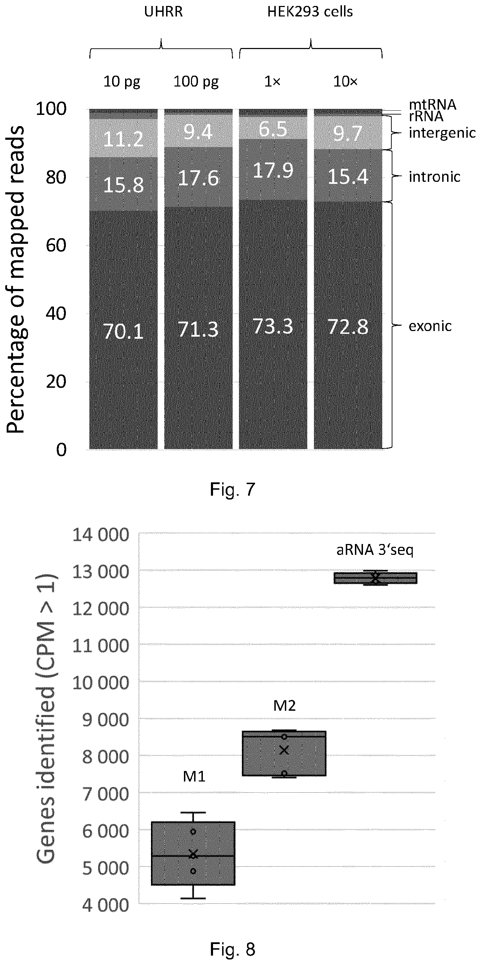

**Figure 7** shows the read distribution statistics after mapping to the annotated human genome, GRCh38.p13. The mapped reads were classified to their supposed origin belonging to exonic and intronic regions of annotated genes, or intergenic regions. The distribution is very conserved and shows a good representation of nascent and matured coding RNA. The reads falling into intergenic regions can be caused by basal transcription, false counting of multimapping reads but also an insufficient annotation.

**Table 1** shows read mapping statistics for a quadruplicate of 1 HEK293 cell each together with a background control which sampled together with inert beads the exact same volume with buffer medium. Primary libraries were amplified to reach enough material for sequencing to analyze the spurious background after the obligatory 3x washes with PBS medium. In the background less than 250 genes were detected, while between 35 and 88% of the reads did not map to the genome at all, and caused by unidentified artefacts. The aRNA-3'seq protocol is highly cell and mRNA specific, and reaches more for a 3'seq protocol more than 75% uniquely mapping reads. The amount of rRNA ranges between 0.1 and 0.2% although no extra rRNA depletion has been applied. The aRNA protocol discriminates very efficiently rRNA and genomic background when applying the method on mRNA template amplification.

**Table 1** aRNA-3'seq NGS reads mapping statistics for experiments in quadruplicates starting with 1 FACS-sorted lysed HEK293 cell or with 1 FACS-sorted inert bead which provide as no-input controls the same buffer volume to quantify any extracellular background RNA which remained in the washed cell culture during the sorting process.

| **FACS RNA source** | **1 HEK293 cell** | | | | **1 bead from the same cell suspension** | | | |
|---|---|---|---|---|---|---|---|---|
| **Replicate** | 1 | 2 | 3 | 4 | 1 | 2 | 3 | 4 |
| **Mapped reads (M)** | 2.4 | 1.9 | 2.0 | 1.9 | 2.1 | 1.9 | 2.1 | 1.9 |
| **Det. genes with CPM >1** | 11 779 | 12 543 | 13 045 | 12 828 | 246 | 169 | 185 | 211 |

| **Proportion of reads mapped (%)** | | | | | | | | |
|---|---|---|---|---|---|---|---|---|
| **Genome** | 97.4 | 97.5 | 97.2 | 97.4 | 64.2 | 11.4 | 22.1 | 27.6 |
| **Uniquely mapped** | 77.5 | 76.3 | 76.5 | 76.2 | 52.7 | 9.0 | 18.2 | 21.1 |
| **Exon** | 70.0 | 70.0 | 68.0 | 69.0 | | | | |
| **Intron** | 24.0 | 24.0 | 25.0 | 25.0 | | | | |
| **Intergenic regions** | 3.4 | 3.5 | 4.2 | 3.4 | | | | |
| **rRNA** | 0.2 | 0.1 | 0.2 | 0.1 | | | | |

The exceptional performance in sensitivity is demonstrated in direct comparison with two other protocols for ultra-low input and single cells, method 1 (M1, NEBNext^{®} Single Cell/Low Input RNA Library Prep Kit for Illumina^{®}, NEB), and method 2 (M2, SMART-seq2, Takara Bio), which represent the current state of the art. In **Figure 8** NGS data sets from experiments with ultra-low input total RNA of 10 pg universal human reference RNA (ThermoFisher Scientific, QS0639) which is representative for the amount of RNA seen in typical cells, were used to calculate comparable gene detection rates. While method M1 detects on average from 6 samples 5,288 genes, M2 could detect already 8,510. However, both are far off the 12,797 genes which were detected with aRNA-3'seq. Important to number of detected genes must be always seen with the read distribution into the classes of gene and intergenic reads where aRNA-3'seq has typically less than 10%. The box plot of **Figure 8** shows also an excellent low variance of the 6 individual samples which is much better than the variance of M1 and M2.

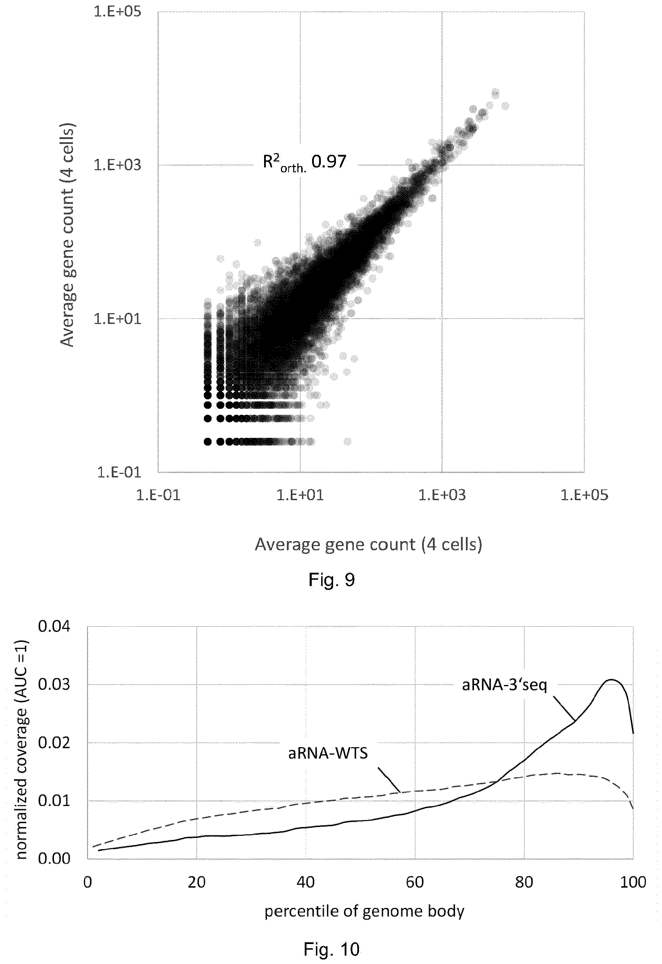

The good reproducibility and wide dynamic range in abundances is shown in **Figure 9****.** The R² value for comparing two expression value medians of 4 individual cells is 0.97.

### Example 4: RNA amplification with PCR-free NGS library preparation

PCR-free library preparation is made by using indexed L1 oligonucleotide probes which also contain the entire Illumina adaptor sequences in oligonucleotide probe L1 and oligonucleotide primer L2. Therefore, no adaptor sequence must be added, and PCR of the transcripts can be omitted. The high degree of linear amplification through IVT generates also enough material, that the pooling and concentrating reaches concentrations of 2 nM ready for entering standard sequencing workflows, e.g., on the NextSeq 500 from Illumina by quantifying the library concentration with standard methods, e.g., Qbit or RT-PCR assay. While single cell library preparations require typically 13 PCR cycles the pooling of 1.9¹³, or 4,205 single cell library preps is sufficient to omit any PCR. Sensitive quantification methods allow also to use lower amounts of NGS library because the subsequent dilution steps can be partially skipped when transferring the pooled concentrated libraries straight into required buffer of the sequencing workflow. For library quantification we recommend RT-PCR.

### Example 5: RNA amplification with whole transcriptome library preparation

The whole transcriptome sequencing (WTS) library preparation used universal human reference RNA (UHRR, Agilent Technologies, Catalog # 740000) as input material. Reverse transcription was carried out in the presence of 0.5 mM free dNTPs, 500 pmol anchored T7pr-oligo(dT)₁₈ probe comprising a T7 promoter sequence and a string of 18 deoxythymidylic acid residues followed by dV, 5'- C TAA TAC GAC TCA CTA TAG GGA GAT TTT TTT TTT TTT TTT TTV-3' (Seq ID NO: 5). RNA-cDNA hybrid molecules were directly treated with single-strand-specific exoribonuclease and DNA polymerase. The end-repair reaction of RNA-cDNA was performed in 50 µl with 20 mM Tris-acetate (pH 7.9 at 25°C), 10 mM Magnesium acetate, 50 mM Potassium acetate, 100 µ/ml BSA, 5 units of single-strand specific exoribonuclease and 3 units of DNA polymerase. Remaining oligonucleotide probes, dNTPs and small fragments were removed by solid phase reverse immobilization (SPRI) with magnetic purification beads (AMPure Beads, Agencourt). The linear amplification by *in vitro* transcription was carried out on the RNA-cDNA hybrids in the presence of 40 mM Tris-HCl (pH 7.9 at 25°C), 6 mM MgCl₂, 10 mM DTT, 10 mM NaCl, 2 mM Spermidine, 200 U T7 RNA polymerase, 3.5 mM rNTPs and 10 % DMSO(at 37°C for 12 hours).

aRNA was purified using 1.8x ratio of SPRI beads, performing the final elution in 10 µl of EB solution (Lexogen GmbH). The purified aRNA was then primed with a random hexamer primer, L2, 5'-C ACG ACG CTC TTC CGA TCT NNN NNN-3' (Seq ID NO: 6), by incubating the primer L2 with the aRNA for 10 min at 25°C, 40 min at 37°C, 10 min at 42°C and 1 min at 25°C with a reverse transcriptase in the presence of RTM buffer (Lexogen GmbH). The hybrids of aRNA with cDNA fragments was then SPRI purified and eluted in 20 µl EB (Lexogen GmbH) before adaptors containing A2 were ligated using the workflow of the Corall whole transcriptome (WTS) library preparation (Lexogen GmbH).

The primary libraries of ssDNA fragments containing both adaptor sequences A1 and A2, or complementary sequences thereof, were amplified in a PCR reaction using a DNA polymerase, PCR buffer and indexed primers (Lexogen GmbH). The PCR was performed by applying 98°C for 30s, followed by cycles between 98°C for 10s, 68°C for 20s, and 72°C for 30s. The optimal number of PCR cycles depends on the RNA input amount and is determined by RT-PCR before starting the endpoint PCR.

In contrast to the aRNA-3'seq were the coverage is concentrated at the 3'ends (**Figure 10**) the present inventive method ("aRNA-WTS") generates reads more evenly distributed across transcripts which is depicted in the averaged overview coverage in **Figure 10****.**

### Example 6: RNA amplification with targeted priming and NGS library preparation

The presented method can be applied to target specific sequences which are either mRNA when using L1 oligonucleotide probes with poly(dT) as complementary sequence, or specific transcripts when using specific complementary sequences in either L1, or L2, or both. Specific complementary sequences can be combined with random sequences to target classes of transcripts like a group of splice variants.

The targeted RNAseq experiment was performed using universal human reference RNA (UHRR, Agilent Technologies, Catalog # 740000) and synthetic RNA SARS-CoV-2 controls (Twist Biosciences, Catalog # SKU:102024). The synthetic SARS RNA was spiked into a background of 10 and 100 ng of UHRR with viral copy (VC) numbers reaching from 2,000 to 20,000 copies per sample. The RNA samples were transferred into lysis buffer (Lexogen GmbH) and heat inactivated (10 min at 50°C, 10 min at 80°C), before further reverse transcribed in the presence of 0.5 mM free dNTPs, 500 pmol SARS-CoV-2 specific oligonucleotide probe L1 comprising a T7 promoter sequence, an adaptor A1, sequence and SARS-CoV-2 specific sequence, 5'-CTA ATA CGA CTC ACT ATA GGG AGA ACG TGT GCT CTT CCG ATC TGT CAT TCT CCT AAG AAG CTA-3' (Seq ID NO: 7). The produced RNA-cDNA hybrid molecules were directly treated with single-strand-specific exoribonuclease and DNA polymerase in 50 ml with 20 mM Tris-acetate (pH 7.9 at 25°C), 10 mM Magnesium acetate, 50 mM Potassium acetate, 100 µ/ml BSA, 5 units of single-strand specific exoribonuclease and 3 units of DNA polymerase. Remaining oligonucleotide probes, dNTPs and small fragments were removed by solid phase reverse immobilization (SPRI) with magnetic purification beads (AMPure Beads, Agencourt). The linear amplification by *in vitro* transcription was carried out on the remaining RNA-cDNA hybrids in the presence of 40 mM Tris-HCl (pH 7.9 at 25°C), 6 mM MgCl₂, 10 mM DTT, 10 mM NaCl, 2 mM Spermidine, 200 U T7 RNA polymerase, 3.5 mM rNTPs and 10 % DMSO at 37°C for 24h. aRNA was purified using 1.8x ratio of SPRI beads, performing the final elution in 10 µl of EB solution (Lexogen GmbH). The purified aRNA was then primed with a random hexamer primer, 5'-C ACG ACG CTC TTC CGA TCT NNN NNN-3' (Seq ID NO: 8), harboring Illumina A2 adaptor sequence. aRNA random priming reaction was incubated for 10 min at 25°C, 40 min at 37°C, 10 min at 42°C and 1 min at 25°C with reverse transcriptase in the presence of RTM buffer (Lexogen GmbH), followed by purification with SPRI beads with a final elution of 20 µl EB (Lexogen GmbH). The obtained primary library was amplified in a PCR reaction using a high-fidelity polymerase and indexed primers (lexogen GmbH). The final amplification was performed by applying 98°C for 30s followed by 16 and 12 cycles, depending on the virus load, of 98°C for 10s, 68°C for 20s, 72°C for 30s. **Figure 11** shows corresponding traces of final cDNA generated from 2,500 and 25,000 viral copies spiked in 10 and 100 ng total UHRR.

### Example 7: aRNA synthesis on indexed beads

Alternatively, to L1 oligonucleotide probes being in solution the reaction can also be initiated by using L1 oligonucleotide probes immobilized on beads. With a pooled-divide synthesis beads with indexed oligonucleotide probes can be synthesized which allow to combine single cells in random manner with single beads which contain one unique sample index each. The design of such functionalized beads in shown in **Figure 12****.** The L1 modified beads can be used in any drop-Seq like analyzes methods in which cells and single beads are combined in tiny volumes or droplets in controlled or random manner in a highly parallel fashion.

The single-cell sequencing method uses a microfluidic device to compartmentalize droplets containing a single cell, lysis buffer, and a microbead covered with indexed probes L1 as shown in **Figure 12****.** Each L1 oligonucleotide probe contains, 1) a complementary poly(dT)V sequence to bind mRNAs, 2) an 8-12 bp unique molecular index (UMI) to identify each mRNA strand uniquely, 3) an 8-12 bp index unique to each cell, and 4) an adaptor sequence A1. Optionally, additional indices can be included which are unique to individual samples, hence identical across all beads used in one experiment. Following compartmen-talization, cells in the droplets are lysed and the released mRNA hybridizes to the oligo(dT)V complementary sequence of the oligonucleotide probe L1 beads. Next, all droplets are pooled and broken to release the beads within. Then, the beads are isolated and brought into an adequate RT buffer (Lexogen GmbH). The bound mRNA is reverse-transcribed using a reverse transcriptase. Next, the products are directly treated with single-strand-specific exoribonuclease and DNA polymerase in 50 µl with 20 mM Tris-acetate (pH 7.9 at 25°C), 10 mM Magnesium acetate, 50 mM Potassium acetate, 100 µ/ml BSA, 5 units of single-strand specific exoribonuclease and 3 units of DNA polymerase. The beads are cleaned to remove dNTPs and exchange the buffer bevor the linear amplification by *in vitro* transcription is carried out on the remaining bead bound RNA-cDNA hybrids in the presence of 40 mM Tris-HCl (pH 7.9 at 25°C), 6 mM MgCl₂, 10 mM DTT, 10 mM NaCl, 2 mM Spermidine, 200 U T7 RNA polymerase, 3.5 mM rNTPs and 10% DMSO at 37°C for 24h. The obtained aRNA is purified using 1.8x ratio of SPRI beads, performing the final elution in 10 µl of EB solution (Lexogen GmbH). The purified aRNA is then primed with a random hexamer primer, 5'-C ACG ACG CTC TTC CGA TCT NNN NNN-3' (Seq ID NO: 9), harboring Illumina A2 linker sequence. aRNA random priming reaction is incubated for 10 min at 25°C, 40 min at 37°C, 10 min at 42°C and 1 min at 25°C with reverse transcriptase in the presence of RTM buffer (Lexogen GmbH), followed by purification with SPRI beads with a final elution of 20 µl EB (Lexogen GmbH). The obtained primary libraries are amplified in a PCR reaction using a high-fidelity polymerase and indexed primers (lexogen GmbH). The final amplification is performed by applying 98°C for 30s followed by 16 and 12 cycles, depending on the virus load, of 98°C for 10s, 68°C for 20s, 72°C for 30s.

The multiple indexed mRNA fragments in the NGS library are ready for sequencing.

**Example 8:** Long-read direct RNA sequencing is possible by using, e.g., Direct RNA Sequencing Kit (SQK-RNA002, Oxford Nanopore Technologies Ltd, UK). In the process, adaptor-modified RNA is sequenced directly without cDNA synthesis and PCR amplification. To reach the required high RNA input amounts the library preparation uses aRNA generated by the inventive method. Then, adaptors harboring an oligo-dT overhang are ligated. The second ligation step incorporates the sequencing adaptors which are preloaded with motor protein that will drive sequencing directly from 3'-poly-A tails to the 5'-cap. Currently, the major limitations of long-read direct sequencing methods are their low throughput (read counts) and high input amount requirements. Previously, direct RNA Sequencing required 500 ng of poly-A RNA. With the inventive method also ultra-low input materials are possible.

The inventive method amplifies mRNA direct from cells or ultra-low input total RNA using poly(dT) containing L1 oligonucleotides to amounts suitable for long-read sequencing. Then, the generated aRNA is polyadenylated at their 3'end using poly(A) Polymerase and buffer containing poly(dA) before the obtained poly(A) aRNA is used as input material for direct RNA sequencing.

## Claims

1. A method of generating transcribed nucleic acids, comprising the steps of
a) providing a nucleic acid template,
b) hybridizing an oligonucleotide probe to the nucleic acid template, wherein said oligonucleotide probe comprises a complementary part that hybridizes to the nucleic acid template and a non-complementary part in 5' direction of the complementary part that does not hybridize to the nucleic acid template and comprises a sequence of a transcription promoter,
c) hydrolysing a 3' part of the nucleic acid template that resides in 3' direction to a part of the nucleic acid template that hybridizes to the oligonucleotide probe in step b) and wherein said 3' part is not hybridized to the oligonucleotide probe,
d) extending the nucleic acid template with nucleic acids complementary to the non-complementary part of the oligonucleotide probe, thereby generating a duplex of the sequence of the transcription promoter in sequence with the nucleic acid template,
e) transcribing the nucleic acid template with a transcriptase that binds the duplex of the sequence of the transcription promoter, thereby generating the transcribed nucleic acids.

2. The method of claim 1, wherein the nucleic acid template comprises or consists of RNA.

3. The method of claim 1 or 2, wherein the hydrolysis is with an exonuclease, preferably a single-stranded RNA specific exonuclease that catalyses the removal of nucleotides in 3'->5' direction.

4. The method of any one of claims 1 to 3, wherein extending the nucleic acid template is by nucleotide polymerisation, preferably with a DNA polymerase.

5. The method of any one of claims 1 to 4, further comprising the step of extending the oligonucleotide probe when hybridized to the nucleic acid template from the complementary part.

6. The method of any one of claims 1 to 5, wherein the non-complementary part of the oligonucleotide probe comprises between the complementary part and the sequence of the transcription promoter an identifier sequence and/or a first adaptor sequence.

7. The method of any one of claims 1 to 6, further comprising hybridizing one or more secondary primers with optionally a second adaptor sequence to the one or more transcribed nucleic acids and extending said secondary primers in a template-dependent manner.

8. The method of any one of claims 1 to 8, wherein the nucleic acid template is RNA from organelles, cell sections, or cells, preferably from 1 to 1000 cells, organelles or cell sections.

9. The method of any one of claims 1 to 8, wherein the nucleic acid template is in a pool of nucleic acids comprising DNA.

10. The method of claim 9 in combination with claim 3, wherein the DNA cannot be digested by the exonuclease of claim 3 and thereby becomes excluded from any subsequent transcription.

11. The method of any one of claims 1 to 10, comprising providing the nucleic acid template in a container and performing steps b) to e) in said container.

12. The method of any one of claims 1 to 11, comprising providing one or more cells, lysing cell material of the cells, inactivating enzymes, preferably by a proteinase, thereby proving nucleic acids of said cells as nucleic acid template according to step a).

13. A set of a plurality of oligonucleotide probes suitable for the method of any one of claims 1 to 12, wherein said oligonucleotide probes each comprises a complementary sequence to a template sequence of choice, preferably comprising a poly(T)-sequence of at least 6 consecutive T's, a transcription promoter sequence and an identifier sequence of at least 4 nucleotides in length, wherein preferably the transcription promoter sequence is the same for the plurality of oligonucleotide probes and/or preferably wherein the identifier sequence is different for at least two oligonucleotide probes of the plurality; and/or preferably wherein the transcription promoter sequence is single stranded.

14. A kit suitable for performing a method of any one of claims 1 to 12, comprising oligonucleotide probes comprising a transcription promoter sequence, a 3'->5' exonuclease, a DNA or RNA polymerase, and a transcriptase capable of initiating transcription at the transcription promoter sequence.

15. The kit of claim 14 further comprising dNTPs, a cell lysis reagent, a proteinase, a reverse transcriptase, or any combination thereof.
